# EUROPEAN PATENT SPECIFICATION

(11) **EP 2 631 642 B1**
(45) Date of publication and mention of the grant of the patent: **19.02.2020**
(21) Application number: 13155940.3
(22) Date of filing: 20.02.2013
(51) Int. Cl.: A61B 8/00, G01N 29/09, G01N 29/24

(54) **Ultrasound apparatus**
Ultraschallvorrichtung
Appareil à ultrasons

(30) Priority: 21.02.2012 JP 2012035159
(43) Date of publication of application: 28.08.2013
(73) Proprietor: Canon Kabushiki Kaisha, Tokyo 146-8501 (JP)
(72) Inventor: Tateyama, Jiro, Tokyo, Tokyo 146-8501 (JP)
(74) Representative: TBK

(56) References cited:
- WO-A1-2008/135896
- WO-A1-2011/055767
- JP-A- H06 217 980
- US-A1- 2007 038 082
- US-A1- 2008 264 171
- US-A1- 2010 232 257
- US-A1- 2011 230 750

## Description

### BACKGROUND OF THE INVENTION

### Field of the Invention

The present invention relates to an ultrasound apparatus.

### Description of the Related Art

As an ultrasound probe for transmitting and receiving an ultrasound wave, a piezoelectric element, such as lead zirconate titanate (PZT), has normally been used. Recently an ultrasound probe using a capacitive micro-machined ultrasonic transducer (CMUT), which is a capacitance type ultrasound converting element, is under research.

A CMUT has a structure that is created using the micro electro mechanical systems (MEMS) process to which a semiconductor process is applied. The CMUT transmits and receives an ultrasound wave using a light vibrating film, and exhibits excellent wide-band characteristics even in liquid or gas. A CMUT is attracting attention since higher precision ultrasound diagnosis than conventional medical image diagnostic modalities can be implemented.

The CMUT is constituted by a combination of a capacitance type ultrasound converting element in the preceding stage and an electric circuit in the subsequent stage, so as to convert a received ultrasound wave into an electric signal (that is, a receiving signal) and output the signal. Here the current of an output signal of the previously mentioned ultrasound converting element is output based on the time variation of the capacitance, therefore a current-voltage conversion amplifying circuit is normally used for the electric circuit in the subsequent stage (see Japanese Patent Application Laid-Open No. 2011-98071).

On the other hand, in the case of an ultrasound diagnostic apparatus that transmits an ultrasound wave to a test object, such as a bio-sample, and images characteristic information of the test object based on a receiving signal acquired by the reflected wave thereof, different scan type probes are used depending on the purpose of diagnosis. Examples of the scan types are: convex, linear, sector and 2-D array. In the same way, using a different ultrasound probe having different transmitting/receiving characteristics, such as PZT and CMUT, is also considered depending on the diagnostic purpose.

The structure normally used for an ultrasound diagnostic apparatus using a probe for PZT is mixing a transmitting signal and a receiving signal for each single element in a connecting unit for connecting the probe, so
as to reduce the number of probe cables by half.

If a probe for CMUT is connected to this connector unit, a protection circuit must be installed for input/output of the current-voltage conversion amplifying circuit which is installed on the receiving side, since voltage higher than the input voltage of the receiving circuit is used for the driving signal on the transmission side. In other words, as the prior art disclosed in Japanese Patent Application Laid-Open No. 2009-297326, the amplifying circuit is protected from overvoltage by using a switch inside the probe in response to a switching control signal at a timing to switch transmission and reception.

However in the case of an apparatus that must change the switching timing for each element individually, the same number of control signals as the number of elements is required, which increases a number of probe cables. Another problem is that the connector unit, constructed for PZT of the ultrasonic diagnostic apparatus, does not include a switching control signal to switch transmission and reception, which means that a dedicated connector unit must be provided separately to connect a probe for CMUT.

In the document US 2011/0230750 A1, there is disclosed a measuring apparatus having: a first probe including a first acoustic conversion element that transmits an ultrasound wave, receives the ultrasound wave after the ultrasound wave is reflected by an object, and converts the ultrasound wave into an analog signal; a second probe including a second acoustic conversion element that receives a photo-acoustic wave generated when light emitted from a light emitter is absorbed by the object and converts the photo-acoustic wave into an analog signal; a receiver that receives the analog signals converted by the first and second acoustic conversion elements and converts the analog signals into digital signals; and a switching unit that switches the acoustic conversion element from which the receiver receives the analog signal.

In the document JP H06 217980 A, there is disclosed an ultrasonic diagnostic device, which aims at miniaturizing a probe for insertion into the body and providing clear tomograms of internal organs. To this end, a switching circuit for supplying transmission high-voltage pulses to a vibrator, a preamplifier disposed near the vibrator, and protective circuits disposed respectively in front of and behind the preamplifier, are all enclosed in a probe. The switching circuit has two diodes connected in series, the threshold voltage of each diode being 2 · VF. The one protective circuit has two diodes and a bias current is supplied to each diode from a DC voltage Vcc via a thick-film resistance. The threshold voltage of each diode in a biased state is + or -VF. The other protective circuit has two diodes and a bias current is supplied to each diode via a thick-film resistance 23e.

Further related art is for example known from the document US 2010/0232257 A1 disclosing an ultrasonic probe and an ultrasonic imaging device, the document WO 2008/135896 A1 disclosing methods and apparatuses of aperture control and multiplexing with adjustable fluid lenses, the document US 2007/0038082 A1 disclosing a medical diagnostic ultrasound transducer system for harmonics, and the document US 2008/0264171 A1 disclosing a reconfigurable array with multilevel transmitters.

### SUMMARY OF THE INVENTION

With the foregoing in view, it is an object of the present invention to provide a technique to operate an ultrasound probe for a piezoelectric element and an ultrasound probe for CMUT using a common cable.

The present invention in its first aspect provides an ultrasound apparatus as specified in claims 1 to 6.

According to the present invention, a technique to operate an ultrasound probe for a piezoelectric element and an ultrasound probe for CMUT using a common cable can be provided.

Further features of the present invention will become apparent from the following description of embodiments with reference to the attached drawings.

### BRIEF DESCRIPTION OF THE DRAWINGS

FIG. 1 is a diagram depicting an overview of a configuration of an ultrasound diagnostic apparatus of the present invention;
FIG. 2 is a diagram depicting a state of connecting a probe for PZT to the ultrasound diagnostic apparatus;
FIG. 3 is a diagram depicting a state of connecting a conventional probe for CMUT to the ultrasound diagnostic apparatus;
FIG. 4 is a diagram depicting an ultrasound diagnostic apparatus according to embodiment 1;
FIG. 5 is a diagram depicting an ultrasound diagnostic apparatus according to embodiment 2; and
FIG. 6 is a diagram depicting an ultrasound diagnostic apparatus according to embodiment 3.

### DESCRIPTION OF THE EMBODIMENTS

Preferred embodiments of the present invention will now be described with reference to the drawings.

The present invention is related to an ultrasound probe for transmitting/receiving an ultrasound wave, and an ultrasound diagnostic apparatus for imaging characteristic information on a test object using a receiving signal acquired by transmitting an ultrasound wave onto a bio-sample.

To be more specific, the apparatus of the present invention uses an ultrasound echo technique that transmits an ultrasound wave from an ultrasound probe to a test object, receives a reflected wave (echo wave) reflected inside the test object, and acquires characteristic information on the inside of the test object as image data. The characteristic information on the inside of the test object acquired here is information reflecting the difference of the acoustic impedance among tissues inside the test object.

The ultrasound wave referred to in this invention is a kind of elastic wave, and includes waves referred to as a sound wave, an ultrasound wave and an acoustic wave.

Typically the ultrasound apparatus according to the present invention is implemented as an ultrasound diagnostic apparatus that acquires characteristic information on a test object, that is a bio-sample, and uses the information for diagnosis. In the following description, the ultrasound apparatus will be explained using such an ultrasound diagnostic apparatus. Also PZT is used as an example of a piezoelectric element that is used for a probe, but a type of piezoelectric element is not limited to PZT. In the following description, "CMUT" refers to a capacitance type ultrasound converting element.

### <Embodiment 1>

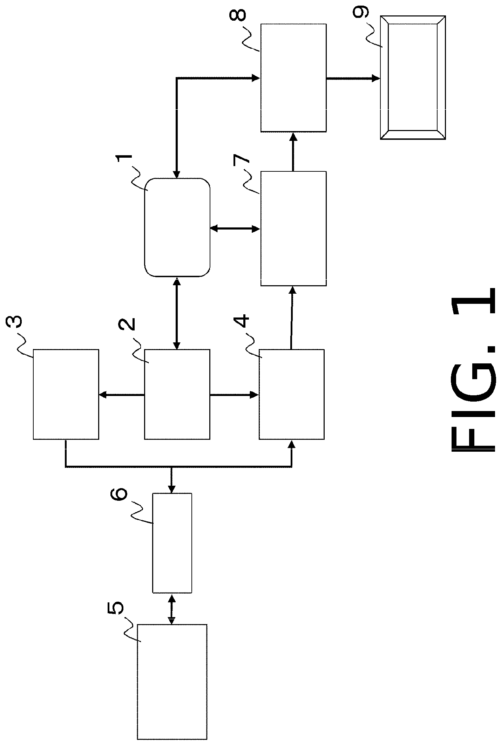

FIG. 1 is a block diagram depicting an ultrasound diagnostic apparatus which represents characteristics of the present invention most clearly. In FIG. 1, the reference numeral 1 denotes a CPU which is a main control of the ultrasound diagnostic apparatus, and the reference numeral 2 denotes a transmission/reception control unit that controls beam forming related to transmission/reception of an ultrasound wave. The reference numeral 5 denotes an ultrasound probe having a structure to generate an ultrasonic wave and detect a reflective echo. The reference numeral 3 denotes a transmitting unit that drives the probe and generates an ultrasound wave, the reference numeral 4 denotes a receiving unit that processes the receiving data detected by the probe, and the reference numeral 6 denotes a signal transmitting cable. The reference numeral 7 is an image processing unit that calculates the form information from the reflective echo wave, the reference numeral 8 denotes a display controlling unit that performs scan conversion, and the reference numeral 9 denotes a display to display an image.

Basic operation for imaging using an ultrasound wave will be described. If an electric signal is transmitted from the transmitting unit 3 in a state of contacting the ultrasound probe 5 to a test object, such as a bio-sample, an ultrasound wave is generated. The ultrasound wave propagates in the object for a very short time, and returns as a reflective echo if the wave contacts something hard. Then the reflective echo is converted into an electric signal by the ultrasound probe 5, and is detected by the receiving unit 4. The detected electric signal becomes a receiving signal. Then the distance from the ultrasound probe to the point where the ultrasound is reflected is calculated on the basis of time from a moment of transmitting the ultrasound wave to a moment of returning the reflective echo, and the image processing unit 7 generates image data on the internal state. Then an image based on the image data is displayed on the display 9 using the display control unit 8, whereby a functional image representing the substance distribution of the bio-tissue can be visualized.

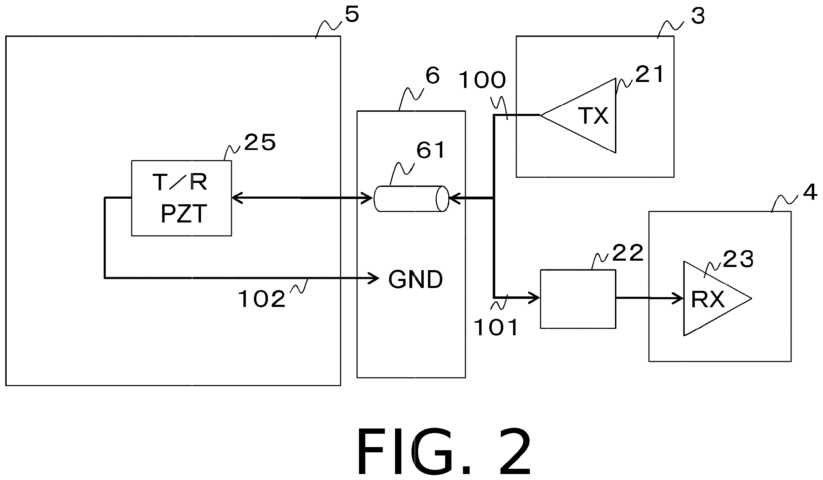

FIG. 2 is a diagram depicting a state of the ultrasound probe 5 for PZT being connected to the apparatus. In FIG. 2, the transmitting unit 3 for driving a transmitting/receiving PZT 25 and generating an ultrasound wave is constructed by a high voltage driving transmitting circuit (TX) 21 constituted by HV-CMOS. The receiving unit 4 uses a receiving unit (RX) 23 to amplify the reflective echo and a weak signal of a photoacoustic wave, which are detected by the probe 5, samples the output by an A/D converter, and performs digital conversion. The receiving unit corresponds to the signal processing unit of the present invention.

A driving signal (transmitting signal) 100 outputted from the transmitting unit 3 becomes a high voltage (e.g. normally about ± 100V) electric signal, which is much higher than an allowable voltage value of a receiving signal 101 which is inputted to the receiving unit. Therefore a limiter 22 must be disposed on the side of input to the receiving circuit. The limiter 22 is normally constituted by a circuit integrating a diode bridge, an inductor and a resistor as discrete components, and has a function to limit the input voltage to the receiving circuit 23 to be within the allowable value.

In the signal transmitting cable 6, a transmitting/receiving signal from the ultrasound probe 5 is connected with a coaxial cable 61, and a GND line 102 which leads to a base of a vibrating element is connected as a separate system. In other words, one coaxial cable corresponds to each vibrating element of PZT, therefore the number of lines of coaxial cables to be used is the same as the number of elements of the probe.

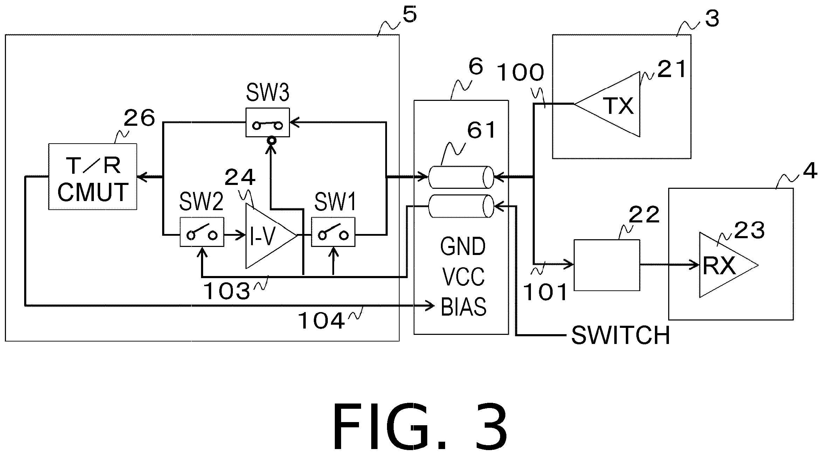

FIG. 3 is a diagram depicting a state of connecting a conventional probe for CMUT to the apparatus. In the probe for CMUT, an I-V conversion amplifying circuit 24 (current-voltage converting circuit) is installed for each element of the transmitting/receiving CMUT 26. The I-V conversion amplifying circuit (current-voltage converting circuit) corresponds to the amplifying unit of the present invention. This means that switch circuits for protecting both the input side and the output side of the I-V conversion amplifying circuit 24 from the driving signal 100 for transmission are required. Therefore switch circuits SW1 and SW2 are installed for the input and the output of the I-V conversion amplifying circuit 24 on the reception side respectively. By turning SW1 and SW2 ON only during the receiving timing period, entry of the driving signal during the transmitting timing period can be prevented. If SW3 is installed on the path on the transmission side, SW3 can be turned OFF during the receiving timing period, and the receiving signal from the CMUT element can be supplied to the I-V conversion amplifying circuit.

A switching control signal (SWITCH) 103 for controlling the ON/OFF of SW1 to SW3 is a signal that is outputted from the transmission/reception control unit 2 which controls beam forming. The receiving timing and the transmitting timing are repeated at a reversed timing, so SW1 and SW2 have a configuration the reverse of the configuration of SW3. This switch circuit is normally constructed by an analog switch when operation speed is considered, but the configuration is not limited to this if the device can switch at high-speed.

If the probe for CMUT is used, a bias power supply (BIAS) 104 must be provided from the outside, therefore a high voltage power supply, about 100 V, is connected as BIAS and operated.

The signal line when the probe for CMUT is connected as shown in FIG. 3, and the signal line when the probe for PZT is connected as shown in FIG. 2 are compared here. In FIG. 3, the transmission/reception switching control signal (SWITCH), the bias power supply for CMUT (BIAS) and VCC which is a driving power supply of the I-V conversion amplifying circuit and the switch circuit, and the GND are added in FIG. 3 to the configuration in FIG. 2.

The transmission/reception control signal is now considered. In the actual ultrasound wave measurement, the transmitting beam forming is often performed, hence a delay is caused to the output timing of the transmitting signal from each element. This means that a timing when high voltage is applied shifts in each element. In this case, the switching control signal must be changed for each element in order to cancel the shift of the timing. In other words, the coaxial cable 61 requires two signal lines for each element.

Based on the above description, Example 1 of the present invention will now be described.

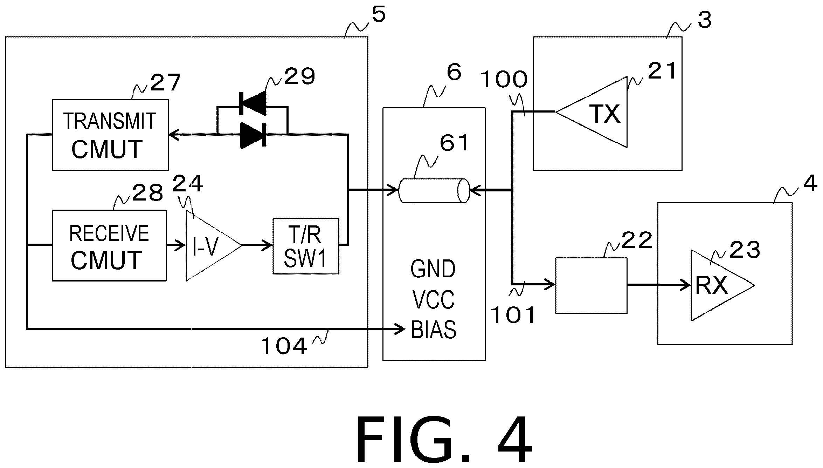

FIG. 4 is a diagram depicting an apparatus according to this example. An ultrasound converting element of the probe for CMUT is characterized in that a transmitting CMUT 27 and a receiving CMUT 28 are constituted by separate CMUTs. The I-V conversion amplifying circuit 24 is installed for each element of the receiving CMUT. If this configuration is used, the protection circuit to protect against the driving signal 100 for transmission can be installed only on the output side of the I-V conversion amplifying circuit. This means that the protection circuit corresponding to SW2 in FIG. 3 is unnecessary, and can be omitted.

In the signal line connecting the receiving CMUT and the I-V conversion amplifying circuit, a signal of weak current outputted from the CMUT flows. Therefore if the SW2 shown in FIG. 3 intervenes, noise is generated in the switch circuit, which causes an increase in noise components at the output end of the amplifying circuit. In the case of embodiment 1 however, SW2 does not exist, so noise components on the reception side can be decreased.

A bidirectional diode 29 is installed on a transmitting path connected to the transmitting CMUT 27. Since only weak signal components during reception can be cut off because of the characteristics of the diode, the function that allows only transmitting signals to pass can be implemented in the same manner as SW3.

A T/R switch (T/R SW1), on the other hand, is a circuit referred to as a high voltage protection device, which protects the output side of the I-V conversion amplifying circuit. The T/R switch means a transmitting/receiving switch, which corresponds to the signal switching unit of the present invention. Unlike the device that turns ON/OFF by the switching control signal used in FIG. 3, this T/R switch is a device that can switch ON/OFF according to the voltage threshold values on both terminals. In other words, the T/R switch can be regarded as a closed switch having a switching resistance that allows a small signal to pass.

The T/R switch of this example starts to turn OFF if the voltage drop between both terminals exceeds ± 2.0V. In the OFF state, this device can withstand ± 100V from the terminal, where only a 200 µA micro-current can flow. The voltage threshold is not limited to this value, but can be a predetermined value according to the characteristics of the apparatus. While a high voltage transmitting signal exceeding the predetermined value is transmitted, the T/R switch enters the OFF state to block the I-V conversion amplifying circuit, and while a transmitting signal is not transmitted, such as a period when a low voltage receiving signal flows, the circuit is connected and the electric signal flows through.

In the signal transmitting cable 6, a transmitting/receiving signal from the ultrasound probe is connected to the coaxial cable 61 for each element. In FIG. 4, the switching control signal (SWITCH) is not used, and only the bias power supply BIAS to supply power to the CMUT and the driving power supply VCC and GND to supply power to the I-V conversion amplifying circuit 24 are connected. In comparison with the signal transmitting cable 6 in the case of using the probe for PZT, shown in FIG. 2, the coaxial cable is commonly used for the transmitting signal and the receiving signal, therefore two types of ultrasound probes can be connected using a common connector. The wiring of VCC and BIAS of the power supply system must be provided separately, which are commonly used by the probe for PZT.

As described above, according to the ultrasound apparatus having the configuration of this example, if a probe for PZT and a probe for CMUT, in which a transmitting element and a receiving element are separate, are alternatively used, a common connector can be used for connecting either probe while protecting circuits from high voltage during transmitting the ultrasound wave. Further, the amount of wiring of the coaxial cable can be reduced.

### <Embodiment 2>

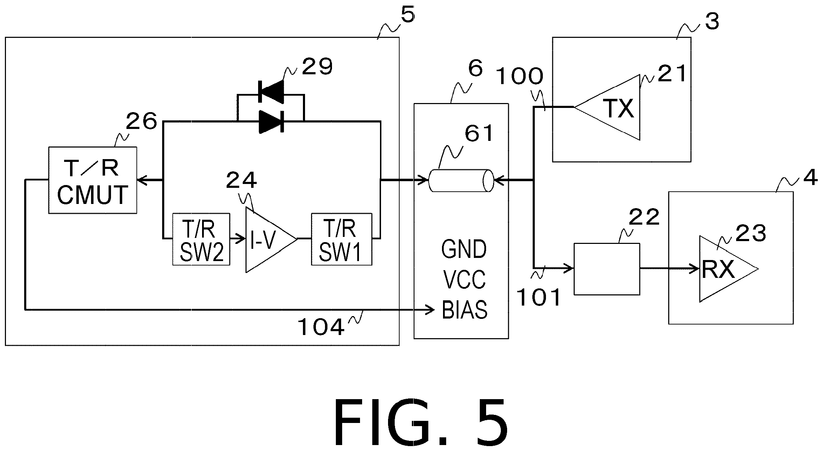

FIG. 5 is a diagram depicting an apparatus according to Example 2 of the present invention. Embodiment 2 is characterized in that the ultrasound converting element of the probe for CMUT is a transmitting/receiving CMUT 26 which is used for both transmission and reception. A bidirectional diode 29 is installed on the path on the transmission side, and an I-V conversion amplifying circuit 24 is installed for each element on the path on the reception side. In this configuration, a protection circuit must be disposed for both the input and output of the I-V conversion amplifying circuit, hence the T/R SW2 is installed on the input side and the T/R SW1 is installed on the output side. In other words, instead of SW1 and SW2 in FIG. 3, the T/R switches are installed, and instead of SW3, the bidirectional diode is installed, whereby the function of the protection switch circuit can be implemented without separately providing a switching control signal.

In the signal transmitting cable 6, a transmitting/receiving signal from the ultrasound probe is connected with the coaxial cable 61 for each element. The switching control signal (SWITCH) is not used, and the bias power supply BIAS to supply power to CMUT and the driving power supply VCC and GND, to supply power to the I-V conversion amplifying circuit 24, are connected. In comparison with the signal transmitting cable 6 in the case of using the probe for PZT shown in FIG. 2, the coaxial cable is commonly used for the transmitting signal and the receiving signal, therefore two types of ultrasound probes can be connected using a common connector. The wiring of the VCC and BIAS of the power supply system must be provided separately, which are commonly used by the probe for PZT.

As described above, according to the ultrasound apparatus having the configuration of this example, a probe for PZT and a probe for CMUT, in which a common element is used for a transmission and reception, are alternatively used, and a common connector can be used for connecting either probe while protecting circuits from high voltage when transmitting the ultrasonic wave. Further, the amount of wiring of the coaxial cable can be reduced.

### <Embodiment 3>

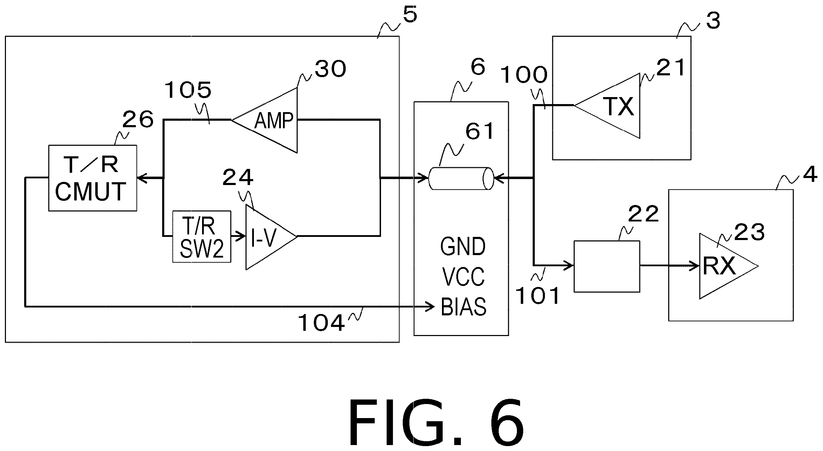

FIG. 6 is a diagram depicting an apparatus according to embodiment 3 of the present invention. The ultrasound converting element of the probe for CMUT is a transmitting/receiving CMUT 26 which is used for both transmission and reception, just like embodiment 2. Embodiment 3 is characterized in that an amplifier (AMP) 30 for voltage conversion is integrated to the path on the transmission side, which can decrease the voltage of the driving signal 100 (transmitting signal) from the transmitting unit. The low voltage driving signal 100 is amplified to be high voltage transmitting signal 105 by the amplifier 30, and is used for ultrasound driving.

In this configuration, high voltage (e.g. ± 100V) is not applied to the coaxial cable 61 for the transmitting/receiving signal in the signal transmitting cable 6, hence the protection circuit need not be installed on the output side of the I-V conversion amplifying circuit. On the input side however, high voltage transmitting signal 105 is applied, hence the T/R SW2 must be installed.

In the signal transmitting cable 6, a transmitting/receiving signal from the ultrasound probe is connected with the coaxial cable 61 for each element. The switching control signal (SWITCH) is not used, and the bias power supply BIAS to supply power to the CMUT and the amplifier, and the driving power supply VCC and GND to supply power to the I-V conversion amplifying circuit 24, are connected. In comparison with the signal transmitting cable 6 in the case of using the probe for PZT shown in FIG. 2, the coaxial cable is commonly used for the transmitting signal and the receiving signal, therefore two types of ultrasound probes can be connected using a common connector. The wiring of the VCC and BIAS of the power supply system must be provided separately, which are commonly used by the probe for PZT.

As described above, according to the ultrasound apparatus having the configuration of this example, if a probe for PZT and a probe for CMUT, in which a common element is used for transmission and reception, are alternatively used, a common connector can be used for connecting either probe while protecting circuits from high voltage when transmitting the ultrasound wave. Further, the amount of wiring of the coaxial cable can be reduced. Furthermore the scale of the protection circuit can be decreased by installing the amplifier.

## Claims

1. An ultrasound apparatus configured to alternatively use a capacitance type ultrasound probe and a piezoelectric type ultrasound probe, comprising:
an ultrasound probe (5) being either one of the capacitance type ultrasound probe and the piezoelectric type ultrasound probe;
a transmitting circuit (3) configured to transmit a transmitting signal, which is an electric signal, to a transmitting unit of a respective one of the capacitance type ultrasound probe and the piezoelectric type ultrasound probe;
a signal processing unit (4) configured to convert a receiving signal, which is received by a receiving unit of a respective one of the capacitance type ultrasound probe and the piezoelectric type ultrasound probe, into a digital signal;
a cable (6) configured to serve as both a path to transmit the transmitting signal from the transmitting circuit to the transmitting unit of a respective one of the capacitance type ultrasound probe and the piezoelectric type ultrasound probe, and a path to transmit the receiving signal from the receiving unit of a respective one of the capacitance type ultrasound probe and the piezoelectric type ultrasound probe to the signal processing unit; and
a common connector configured to connect either ultrasound probe of the capacitance type ultrasound probe and the piezoelectric type ultrasound probe to the cable,
wherein the piezoelectric type ultrasound probe includes:
a transmitting unit (25) configured to transmit an ultrasound wave in response to an input of the transmitting signal, and
a receiving unit (25) configured to generate the receiving signal by converting a reflected wave of the transmitted ultrasound wave into an electric signal; and
wherein the capacitance type ultrasound probe includes:
a transmitting unit (26, 27) configured to transmit an ultrasound wave in response to an input of the transmitting signal via a transmission path of the capacitance type ultrasound probe,
a receiving unit (26, 28) configured to generate the receiving signal by converting a reflected wave of the transmitted ultrasound wave into an electric signal on a reception path of the capacitance type ultrasound probe, and
an amplifying unit (24) configured to amplify the receiving signal, which is installed on the reception path of the capacitance type ultrasound probe; and
wherein the capacitance type ultrasound probe further includes:
a signal switching unit configured to interrupt the amplifying unit from a part of the reception path, through which the transmitting signal passes, while the ultrasound wave is being transmitted, wherein
the signal switching unit is installed on the reception path of the capacitance type ultrasound probe, and
the signal switching unit is configured to detect a voltage value of the transmitting signal, and to interrupt the amplifying unit when the voltage value exceeds a predetermined value.

2. The ultrasound apparatus according to Claim 1, wherein
the transmitting unit and the receiving unit of the capacitance type ultrasound probe are capacitance type ultrasound converting elements.

3. The ultrasound apparatus according to Claim 1 or 2, wherein
the amplifying unit of the capacitance type ultrasound probe is a current-voltage converting circuit.

4. The ultrasound apparatus according to any one of Claims 1 to 3, wherein
the transmitting unit and the receiving unit of the capacitance type ultrasound probe are constituted by different capacitance type ultrasound converting elements (27, 28) respectively, and
the signal switching unit is installed on the output side of the amplifying unit (24) on the reception path of the capacitance type ultrasound probe.

5. The ultrasound apparatus according to any one of Claims 1 to 3, wherein
a same capacitance ultrasound converting element (26) serves as both the transmitting unit and the receiving unit of the capacitance type ultrasound probe, and
the signal switching unit is installed on the input side and the output side of the amplifying unit (24) on the reception path of the capacitance type ultrasound probe.

6. The ultrasound apparatus according to any one of Claims 1 to 3, wherein
a same capacitance type ultrasound converting element (26) serves as both the transmitting unit and the receiving unit of the capacitance type ultrasound probe,
an amplifier (30) for amplifying the transmitting signal is further included on the transmission path of the capacitance type ultrasound probe, and
the signal switching unit is installed on the input side of the amplifying unit (24) on the reception path of the capacitance type ultrasound probe.

## Patentansprüche

1. Ultraschallvorrichtung, die konfiguriert ist zur alternativen Verwendung eines Ultraschallkopfs kapazitiven Typs und eines Ultraschallkopfs piezoelektrischen Typs, mit:
einem Ultraschallkopf (5), der entweder der Ultraschallkopf kapazitiven Typs oder der Ultraschallkopf piezoelektrischen Typs ist;
einer Sendeschaltung (3), die konfiguriert ist zum Senden eines Sendesignals, das ein elektrisches Signal ist, an eine Sendeeinheit von einem jeweiligen des Ultraschallkopfs kapazitiven Typs und des Ultraschallkopfs piezoelektrischen Typs;
einer Signalverarbeitungseinheit (4), die konfiguriert ist zum Wandeln eines Empfangssignals, das durch eine Empfangseinheit von einem jeweiligen des Ultraschallkopfs kapazitiven Typs und des Ultraschallkopfs piezoelektrischen Typs empfangen wird, in ein digitales Signal;
einem Kabel (6), das konfiguriert ist zum Dienen als sowohl ein Pfad zum Senden des Sendesignals von der Sendeschaltung an die Sendeeinheit von einem jeweiligen des Ultraschallkopfs kapazitiven Typs und des Ultraschallkopfs piezoelektrischen Typs als auch ein Pfad zum Senden des Empfangssignals von der Empfangseinheit von einem jeweiligen des Ultraschallkopfs kapazitiven Typs und des Ultraschallkopfs piezoelektrischen Typs an die Signalverarbeitungseinheit; und
einer gemeinsamen Verbindungseinrichtung, die konfiguriert ist zum Verbinden von einem Ultraschallkopf von dem Ultraschallkopf kapazitiven Typs und dem Ultraschallkopf piezoelektrischen Typs mit dem Kabel,
wobei der Ultraschallkopf piezoelektrischen Typs umfasst:
eine Sendeeinheit (25), die konfiguriert ist zum Senden einer Ultraschallwelle in Erwiderung auf eine Eingabe des Sendesignals, und
eine Empfangseinheit (25), die konfiguriert ist zum Erzeugen des Empfangssignals durch Wandeln einer reflektierten Welle der gesendeten Ultraschallwelle in ein elektrisches Signal; und
wobei der Ultraschallkopf kapazitiven Typs umfasst:
eine Sendeeinheit (26, 27), die konfiguriert ist zum Senden einer Ultraschallwelle in Erwiderung auf eine Eingabe des Sendesignals über einen Sendepfad des Ultraschallkopfs kapazitiven Typs,
eine Empfangseinheit (26, 28), die konfiguriert ist zum Erzeugen des Empfangssignals durch Wandeln einer reflektierten Welle der gesendeten Ultraschallwelle in ein elektrisches Signal auf einem Empfangspfad des Ultraschallkopfs kapazitiven Typs, und
eine Verstärkungseinheit (24), die konfiguriert ist zum Verstärken des Empfangssignals, wobei diese auf dem Empfangspfad des Ultraschallkopfs kapazitiven Typs eingerichtet ist; und
wobei der Ultraschallkopf kapazitiven Typs zusätzlich umfasst:
eine Signalschalteinheit, die konfiguriert ist zum Trennen der Verstärkungseinheit von einem Teil des Empfangspfads, über den das Sendesignal verläuft, während die Ultraschallwelle gesendet wird, wobei
die Signalschalteinheit auf dem Empfangspfad des Ultraschallkopfs kapazitiven Typs eingerichtet ist, und
die Signalschalteinheit konfiguriert ist zum Detektieren eines Spannungswerts des Sendesignals und zum Trennen der Verstärkungseinheit, wenn der Spannungswert einen vorbestimmten Wert überschreitet.

2. Ultraschallvorrichtung gemäß Anspruch 1, wobei
die Sendeeinheit und die Empfangseinheit des Ultraschallkopfs kapazitiven Typs Ultraschallwandlungselemente kapazitiven Typs sind.

3. Ultraschallvorrichtung gemäß Anspruch 1 oder 2, wobei
die Verstärkungseinheit des Ultraschallkopfs kapazitiven Typs eine Strom-Spannung-Wandlungsschaltung ist.

4. Ultraschallvorrichtung gemäß einem der Ansprüche 1 bis 3, wobei
die Sendeeinheit und die Empfangseinheit des Ultraschallkopfs kapazitiven Typs jeweils durch verschiedene Ultraschallwandlungselemente kapazitiven Typs (27, 28) gebildet sind, und
die Signalschalteinheit auf der Ausgangsseite der Verstärkungseinheit (24) auf dem Empfangspfad des Ultraschallkopfs kapazitiven Typs eingerichtet ist.

5. Ultraschallvorrichtung gemäß einem der Ansprüche 1 bis 3, wobei
ein gleiches Ultraschallwandlungselement kapazitiven Typs (26) als sowohl die Sendeeinheit als auch die Empfangseinheit des Ultraschallkopfs kapazitiven Typs dient, und
die Signalschalteinheit auf der Eingangsseite und der Ausgangsseite der Verstärkungseinheit (24) auf dem Empfangspfad des Ultraschallkopfs kapazitiven Typs eingerichtet ist.

6. Ultraschallvorrichtung gemäß einem der Ansprüche 1 bis 3, wobei
ein gleiches Ultraschallwandlungselement kapazitiven Typs (26) als sowohl die Sendeeinheit als auch die Empfangseinheit des Ultraschallkopfs kapazitiven Typs dient,
ein Verstärker (30) zum Verstärken des Sendesignals zusätzlich auf dem Sendepfad des Ultraschallkopfs kapazitiven Typs umfasst ist, und
die Signalschalteinheit auf der Eingangsseite der Verstärkungseinheit (24) auf dem Empfangspfad des Ultraschallkopfs kapazitiven Typs eingerichtet ist.

## Revendications

1. Appareil à ultrasons configuré pour utiliser en alternance une sonde ultrasonore de type capacitif et une sonde ultrasonore de type piézoélectrique, comprenant :
une sonde ultrasonore (5) qui est l'une ou l'autre de la sonde ultrasonore de type capacitif et de la sonde ultrasonore de type piézoélectrique ;
un circuit de transmission (3) configuré pour transmettre un signal de transmission, qui est un signal électrique, à une unité de transmission de l'une respective de la sonde ultrasonore de type capacitif et de la sonde ultrasonore de type piézoélectrique ;
une unité de traitement de signal (4) configurée pour convertir un signal de réception, qui est reçu par une unité de réception de l'une respective de la sonde ultrasonore de type capacitif et de la sonde ultrasonore de type piézoélectrique, en un signal numérique ;
un câble (6) configuré pour servir à la fois de trajet de transmission du signal de transmission du circuit de transmission à l'unité de transmission de l'une respective de la sonde ultrasonore de type capacitif et de la sonde ultrasonore de type piézoélectrique, et de trajet de transmission du signal de réception de l'unité de réception de l'une respective de la sonde ultrasonore de type capacitif et de la sonde ultrasonore de type piézoélectrique à l'unité de traitement de signal ; et
un connecteur commun configuré pour connecter l'une ou l'autre de la sonde ultrasonore de type capacitif et de la sonde ultrasonore de type piézoélectrique au câble,
dans lequel la sonde ultrasonore de type piézoélectrique comprend :
une unité de transmission (25) configurée pour transmettre une onde ultrasonore en réponse à une entrée du signal de transmission, et
une unité de réception (25) configurée pour générer le signal de réception par une conversion d'une onde réfléchie de l'onde ultrasonore transmise en un signal électrique ; et
dans lequel la sonde de type capacitif comprend :
une unité de transmission (26, 27) configurée pour transmettre une onde ultrasonore en réponse à une entrée du signal de transmission par le biais d'un trajet de transmission de la sonde ultrasonore de type capacitif,
une unité de réception (26, 28) configurée pour générer le signal de réception par une conversion d'une onde réfléchie de l'onde ultrasonore transmise en un signal électrique sur un trajet de transmission de la sonde ultrasonore de type capacitif, et
une unité d'amplification (24) configurée pour amplifier le signal de réception, qui est installée sur le trajet de réception de la sonde ultrasonore de type capacitif ; et
dans lequel la sonde ultrasonore de type capacitif comprend en outre :
une unité de commutation de signal configurée pour couper l'unité d'amplification d'une partie du trajet de réception, par l'intermédiaire de laquelle passe le signal de transmission, tandis que l'onde ultrasonore est en cours de transmission, où
l'unité de commutation de signal est installée sur le trajet de réception de l'onde ultrasonore de type capacitif, et
l'unité de commutation de signal est configurée pour détecter une valeur de tension du signal de transmission, et pour couper l'unité d'amplification lorsque la valeur de tension dépasse une valeur prédéterminée.

2. Appareil à ultrasons selon la revendication 1, dans lequel
l'unité de transmission et l'unité de réception de la sonde ultrasonore de type capacitif sont des éléments de conversion d'ultrasons de type capacitif.

3. Appareil à ultrasons selon la revendication 1 ou 2, dans lequel
l'unité d'amplification de la sonde ultrasonore de type capacitif est un circuit de conversion courant-tension.

4. Appareil à ultrasons selon l'une quelconque des revendications 1 à 3, dans lequel
l'unité de transmission et l'unité de réception de la sonde ultrasonore de type capacitif sont constituées respectivement par des éléments de conversion d'ultrasons de type capacitif différents (27, 28), et
l'unité de commutation de signal est installée du côté sortie de l'unité d'amplification (24) sur le trajet de réception de la sonde ultrasonore de type capacitif.

5. Appareil à ultrasons selon l'une quelconque des revendications 1 à 3, dans lequel
un même élément de conversion d'ultrasons de type capacitif (26) sert à la fois d'unité de transmission et d'unité de réception de la sonde ultrasonore de type capacitif, et
l'unité de commutation de signal est installée du côté entrée et du côté sortie de l'unité d'amplification (24) sur le trajet de réception de la sonde ultrasonore de type capacitif.

6. Appareil à ultrasons selon l'une quelconque des revendications 1 à 3, dans lequel
un même élément de conversion d'ultrasons de type capacitif (26) sert à la fois d'unité de transmission et d'unité de réception de la sonde ultrasonore de type capacitif,
un amplificateur (30) destiné à amplifier le signal de transmission est en outre compris sur le trajet de transmission de la sonde ultrasonore de type capacitif, et
l'unité de commutation de signal est installée du côté entrée de l'unité d'amplification (24) sur le trajet de réception de la sonde ultrasonore de type capacitif.
